# EUROPEAN PATENT SPECIFICATION

(11) **EP 0 309 263 B1**
(45) Date of publication and mention of the grant of the patent: **09.03.1994**
(21) Application number: 88308840.3
(22) Date of filing: 23.09.1988
(51) Int. Cl.: A61K 31/565

(54) **Hormone composition and use**
Hormon-Zusammensetzung und -Anwendung
Composition d'hormones et application

(30) Priority: 24.09.1987 CA 547743; 24.09.1987 CA 547744
(43) Date of publication of application: 29.03.1989
(60) Divisional application: 93107794.5
(73) Proprietor: JENCAP RESEARCH LIMITED, Toronto, Ontario M5R 2Y4 (CA)
(72) Inventor: Casper, Robert F., 6-240 Eaton North, Toronto, Ontario M5G 2C4 (CA)
(74) Representative: Jones, Helen Marjorie Meredith

(56) References cited:
- EP-A- 0 253 607
- DE-A- 2 253 916
- DE-A- 2 645 307
- FR-A- 2 589 735
- GB-A- 2 096 462
- US-A- 3 568 828
- US-A- 4 291 028
- US-A- 4 292 315
- Rote Liste, 1987, Editio Cantor, Aulendorf, Württemberg, DE, no. 75 157
- Rote Liste, 1987, Editio Cantor, Aulendorf, Württemberg, DE, no. 75 154, no. 75 156
- Unlisted Drugs., vol. 38, no. 1, January 1986, p. 2, Chatham, N.J., US
- Chemical Abstracts, vol. 83, 1975, no. 72528q
- Med. Akt. 8 (1982), p. 418
- New England J. of Medicine, 304 (March 5) 1981, pp. 560-563
- Unlisted Drugs, vol. 22, no. 10, October 1970, p. 149
- Unlisted Drugs, vol. 25, no. 10, October 1973, p. 160
- Unlisted Drugs, vol. 26, no. 11, November 1974, p. 170
- Unlisted Drugs, vol. 28, no. 2, Febr. 1976, p. 26
- Current thep. Res. 12 (1970) 4, 177 & WB 1970, 11, 20

## Description

This invention is concerned with a hormone replacement formulation which employs a combination of estrogen and progestin and wherein a short period of relatively dominant estrogenic activity alternates with a short period of relatively dominant progestagenic activity for hormonal replacement therapy for menopausal or castrate women.

In the luteal phase of the menstrual cycle, serum progesterone levels increase and progesterone mediated secretory changes occur in the uterine endometrium. The presence of progesterone receptors has been shown to be a necessary prerequisite for progesterone action in the endometrium (see Walters, M.R. and Clark, J.H. Relationship between the quantity of progesterone receptors and the antagonism of estrogen-induced uterotropic response Endocrinology 105:382, 1979) and it is well documented that estrogen priming in the follicular phase of the cycle is responsible for the development of both estrogen and progesterone receptors (see Bayard, F., Damilano, S., Robel, P. and Baulien, E.E. Cytoplasmic and nuclear estradiol and progesterone receptors in human endometrium, J. Clin Endocrinol Metab. 46:635, 1978). On the other hand, progesterone exerts a negative feedback effect on its own receptor (see Tseng, L. and Gurpide, E. Effects of progestins on estradiol receptor levels in human endometrium, J. Clin Endocrinol Metab. 41:402, 1975) and also acts to downregulate endometrial estrogen receptors possibly by induction of an estrogen receptor regulatory factor (see Leavitt, W.W., Okulicz, W.C., McCracken, J.A., Schramm, W.S. and Robidoux, W.F. Jr. Rapid recovery of nuclear estrogen receptor and oxytocin receptor in the ovine uterus following progesterone withdrawal, J. Steroid Biochem. 22:686, 1985).

These physiologic changes can be reproduced pharmacologically as shown by the induction of estrogen and progestin receptors in postmenopausal women by the administration of ethinyl estradiol (see Kreitmann, B., bugat, R. and Bayard, F. Estrogen and Progestin Regulation of the Progesterone Receptor Concentration in Human Endometrium, J. Clin Endocrinol Metab. 49:926, 1979). Neumannova et al (see Short-Term Effects of Tamoxifen, Medroxy-progesterone Acetate, and Their Combination on Receptor Kinetics and 17beta-Hydroxysteroid Dehydrogenase in Human Endometrium, Obstet. Gynecol. 66:695, 1985) have also demonstrated that administration of medroxy-progesterone acetate in estrogen-primed women decreases the concentration of endometrial progestin receptors while at the same time increasing the activity of 17beta-hydroxysteroid dehydrogenase, an enzyme which is responsible for metabolism of estradiol to the less potent estrone.

A complex interaction occurs between estrogen and progesterone or progestin in the human endometrium with the progestins acting as anti-estrogens. Estrogen and progestin interactions are also dynamic. For example, estrogen administration increased the concentration of both estrogen and progestin receptors to peak levels, 7 times above baseline, within 3 days (see Ekert, R.L. and Katzenellenbogen, B.S. Human Endometrial Cells in Primary Tissue Culture: Modulation of the Progesterone Receptor Level by Natural and Synthetic Estrogens In Vitro, J. Clin Endocrinol Metab. 52:699, 1981). A three-fold increase in receptor concentrations occurred within one day. Normal physiologic levels of progesterone in the first 3 days of the luteal phase resulted in a rapid and significant decrease in estrogen receptor number (see Kreitmann-Gimbal, B., Bayard, F., Nixon, W.E. and Hodgen, G.D. Patterns of Estrogen and Progesterone Receptors in Monkey Endometrium During the Normal Menstrual Cycle, Steroids 35:471, 1980). Exogenous administration of progesterone to cynomolgous macaques significantly suppressed estrogen receptors within 1 to 2 days (see West, N.B. and Brenner, R.M. Progesterone-Mediated Suppression of Estradiol Receptors in Cynomolgous Macaque Cervix, Endometrium and Oviduct During Sequential Estradiol-Progesterone Treatment, J. Steroid Biochem. 22:29, 1985) and medroxy-progesterone acetate was able to significantly suppress progestin receptor levels in premenopausal women within 4 hours (see Neumannova M., Kauppila, A., Kivinen, S. and Vihko, R. Short-Term Effects of Tamoxifen, Medroxy-progesterone Acetate, and Their Combination on Receptor Kinetics and 17beta-Hydroxysteroid Dehydrogenase in Human Endometrium, Obstet. Gynecol. 66:695, 1985). In contrast, progesterone withdrawal in the presence of constant estrogen levels has been shown to result in rapid (6 to 12 hours) recovery of nuclear estrogen receptors in sheep endometrium, associated with an estrogen induced biological response, i.e. production of oxytocin receptors (see Leavitt, W.W., Okulicz, W.C., McCracken, J.A., Schramm, W.S. and Robidoux, W.F. Jr. Rapid recovery of nuclear estrogen receptor and oxytocin receptor in the ovine uterus following progesterone withdrawal, J. Steroid Biochem. 22:686, 1985). A similar phenomenon occurs in pregnant guinea pigs when estrogen levels rise relative to progesterone levels prior to parturition (see Alexandrova, M. and Soloff, M.S. Oxytocin receptors and parturition in the guinea pig, Biol. Reprod. 22:1106, 1980).

Therefore, it appears that estrogen acts to stimulate both estrogen and progestin receptor concentrations and to induce sensitivity of the endometrium to both estrogen and progestin. Progesterone or progestin exerts an anti-estrogen action by decreasing estrogen receptors and by increasing 17beta-hydroxysteroid dehydrogenase activity in endometrial tissue. However, it appears that the stimulatory effects of progesterone on human endometrial function are of short duration probably because of a self-provoked downregulation of progestin receptors and estrogen receptors. For example, the effect of progesterone on 17beta-hydroxysteroid dehydrogenase peaks at 3 days and is then followed in 2 to 3 weeks by suppression of the enzyme (see Whitehead, M.I., Townsend, P.T., Pryse-Davies, J. et al. Effects of estrogens and progestins on the biochemistry and morphology of the postmenopausal endometrium, N. Engl, J. Med 305:1599, 1981).

Currently on the market there are a number of contraceptive formulations which can be classified readily into several general types. The first of these are known as monophasic formulations. These contain a constant amount of estrogen and progestin. Nuisance side effects with these pills depend on the balance between the estrogen and progestin component of the pill. For example, with a relatively dominant progestin pill, the formulation will, over time, result in a depletion of both estrogen and progestin receptors. The result which might be expected is an understimulated or atrophic endometrium which may eventually cause either on-pill amenorrhea or breakthrough bleeding or spotting due to poor epithelialization. On the other hand, with a relatively dominant estrogenic preparation, it is possible that prolonged use could result in endometrial growth with the development of unsupported fragile stroma and subsequent spotting or breakthrough bleeding.

Newer formulations known as triphasics have varying levels of estrogen and progestin; in most cases consisting of relatively constant levels of estrogen with a step-wise increase in progestin throughout the cycle. This pattern of estrogen and progestin administration results in a relatively dominant estrogenic formulation at the beginning of the package with increasing progestagenic activity toward the end of the package. Endometrial stability may be better with these pills since the estrogenic activity at the beginning of the package induces both estrogen and progestin receptors making the endometrium sensitive to the increased levels of progestin towards the end of the package. The progestin activity produces denser, more stable endometrial stroma although the relatively long duration of progestin exposure, toward the end of the package, may still lead to decreased estrogen and progestin receptors and activity. A significant problem with this type of formulation is the low dose of steroids at the beginning of the package which makes these pills vulnerable to drug interactions or missed pills which may lead to breakthrough ovulation. The beginning of the package is the critical time in terms of breakthrough ovulation since the user has just completed a 7 day drug-free interval during which follicular development may begin. Even if pregnancy does not occur, breakthrough ovulation can lead to poor cycle control.

Estrogen replacement therapy is warranted in menopausal women for several reasons. Estrogen replacement will relieve hot flushes and this relief of flushes and night sweats improves sleep patterns and contributes to the patient's general feeling of well-being (see Campbell S., Whitehead M.I. Estrogen therapy and the menopausal syndrome. In Clinics in Obstetrics and Gynecology: Volume 4. The Menopause. Edited by R.B. Greenblatt, J.W.W. Studd, London, W.B. Saunders, 1977, pages 31-47; Erlik Y., Tataryn I.V., Meldrum D.R. et al. Association of waking episodes with menopausal hot flushes. JAMA 24:1741, 1981). Estrogen replacement protects against postmenopausal loss of calcium from the skeleton, especially from vertebral bodies, preventing crush fractures and loss of body height (see Lindsay R., Hart D.M., Forrest, C. et al. Prevention of spinal osteoporosis in oophorectomized women. Lancet 2:1151, 1980). Several studies have now reported that long-term estrogen therapy is also associated with a reduction in the incidence of classical osteoporotic fractures of the forearm and hip (see Hutchinson, T.A., Polansky, S.M., Finestein, A. Postmenopausal estrogens protect against fractures of hip and distal radius. Lancet 2:706, 1979; Paganini-Hill, A., Ross, R.K., Gerkins, V.R., et al. A case control study of menopausal estrogen therapy in hip fractures. Annals of Internal Medicine 95:28, 1981; Weiss N.S., Ure C.L., Ballard J.H. et al. Decreased risk of fractures of the hip and lower forearm with postmenopausal use of estrogen. New England Journal of Medicine 303:1195, 1980). Another beneficial effect of long-term estrogen use is the reduction of the risk of death from ischemic heart disease probably mediated by changes in blood lipoprotein concentrations (see Ross R.K., Paganini-Hill A., Mack T.M. et al. Menopausal estrogen therapy and protection from ischemic heart disease. Lancet 1:858, 1981). Estrogen replacement has also been shown to improve the vascularity and health of the vaginal mucosa and urinary tract. The only major risk factor associated with estrogen administration in the doses required to relieve menopausal symptoms, is hyperstimulation of the endometrium and an increased risk of endometrial cancer (see Cramer D.W., Knapp R.C. Review of epidemiologic studies of endometrial cancer and exogenous estrogen. Obstetrics and Gynecology 54:521, 1979; Shapiro S., Coughman D.W., Sloan D., et al. Recent and past use of conjugated estrogens in relation to adenocarcinoma of the endometrium. New England Journal of Medicine 303:485, 1980).

Estrogens predispose to cancer of the endometrium by stimulating cell mitosis and proliferation and increasing the levels of DNA synthesis and nuclear estradiol receptors in the endometrium (see Whitehead M.I., Townsen P.T., Pryce-Davies J., et al. Effects of estrogens and progestins on the biochemistry and morphology of the postmenopausal endometrium. New England Journal of Medicine 305:599, 1981; Whitehead M.I., Townsen P.T., Pryce-Davies J., et al. Actions of progestins on the morphology and biochemistry of the endometrium of postmenopausal women receiving low dose estrogen therapy. American Journal of Obstetrics and Gynecology, 142:791, 1982).

The addition of a progestin for 13 days each month has been demonstrated to protect the endometrium from these stimulatory effects of estrogen (see Gambrill R.D., Jr., Massey F.M., Castaneda et al. Use of the progestogen challenge test to reduce the risk of endometrial cancer. Obstetrics and Gynecology 55:732, 1980; Studd J.W.W., Thom M.H., Patterson M.E.L., Wade-Evans T. The prevention and treatment of endometrial pathology in postmenopausal women receiving exogenous estrogens. In: Pasetto N., Paoletti R., Armbus J.L., Editors. The menopause and postmenopause. Lancester MPT Press. 127,1980).

The addition of a progestin protects the endometrium by reducing nuclear estradiol receptor concentration and thereby decrease nuclear estrogen bioavailability resulting in an antimitotic effect and lowering DNA synthesis. Progestins also increase the activity of endometrial estradiol-l7beta-dehydrogenase, an enzyme which metabolizes estradiol to estrone, a less potent estrogen (see Whitehead M.I., Townsen P.T., Pryce-Davies J. Effects of estrogens and progestins on the biochemistry and morphology of the postmenopausal endometrium. New England Journal of Medicine. 305:1599, 1981; King R.J.B., Townsen P.T., Sittle N.C., et al. Regulation of estrogen and progesterone receptor levels in epithelium and stroma from pre and postmenopausal endometria. Journal of Steroids and Biochemistry, 16:21, 1982; Gurpide E. Enzymatic modulation of hormonal action at the target tissue. Journal of Toxicology and Environmental Health, 4:249, 1978). The addition of progestin to estrogen replacement therapy may also result in an increase in bone mass when started within 3 years of the menopause (see Nachtigall L.E., Nachtigall R.H., Nachtigall R.D., et al. Estrogen replacement therapy: A 10 year prospective study in relationship to osteoporosis. Obstetrics and Gynecology 53:277, 1979; Lindsay R., Hart D.M., Forrest C., et al. Prevention of spinal osteoporosis in oophorectomized women. Lancet 2:1151, 1980). However, concerns have been expressed about the potential adverse effects of progestin in suppressing high density lipoprotein cholesterol concentrations (see Hirvonen E., Malkonen M., Manninen V. Effects of different progestogens on lipoproteins during postmenopausal replacement therapy. New England Journal of Medicine 304:560, 1981). This cholesterol fraction appears to have a protective effect against ischemic heart disease and atherosclerosis. The lowering of HDL cholesterol by progestin could negate the long-term beneficial effects of estrogen in reducing the incidence of myocardial infraction. Other side effects of progestins include acne, breast tenderness, depression and irritability (see Barranco V.P. Effect of androgen dominant and estrogen dominant oral contraceptives on acne. Cutis 14:384, 1974; Royal College of General Practioners. Oral Contraceptives and Health: An Interim Report. New York: Pitman, 1974). Since the side effects of progestins appear to be dose dependent, the dose of progestin used with postmenopausal estrogen replacement should be the minimum necessary to achieve endometrial protection. (see Padwick M.L., Pryce-Davies J., Whitehead M.I. A simple method for determining the optimal dosage of progestin in postmenopausal women receiving estrogens. New England Journal of Medicine 315:930, 1986).

The biological effects of both estrogen and progestin in target tissues such as the endometrium are dependent on the levels of estrogen and progestin receptors. Both estrogen and progestins exert a modulating influence on the levels of their own receptors. For example, in the luteal phase of the menstrual cycle, serum progesterone levels increase and progesterone mediated secretory changes occur in the uterine endometrium. The presence of progesterone receptors has been shown to be a necessary prerequisite for progesterone action in the endometrium (see Walters M.R. and Clark J.H. Relationship between the quantity of progesterone receptors and the antagonism of estrogen-induced uterotropic response. Endocrinology 105:382, 1979) and it is well documented that estrogen priming in the follicular phase of the cycle is responsible for the development of both estrogen and progesterone receptors (see Bayard F., Damilano S., Robel P. and Baulieu E.E. Cytoplasmic and nuclear estradiol and progesterone receptors in human endometrium. Journal Clinical Endocrinology and Metabolism 46:635, 1978). On the other hand, progesterone exerts a negative feedback effect on its own receptor (see Tseng L. and Gurpide E. Effects of progestins on estradiol receptor levels in human endometrium. Journal Clinical Endocrinology and Metabolism 41:402, 1975) and also acts to downregulate endometrial estrogen receptors possibly by induction of an estrogen receptor regulatory factor (see Leavitt W.W., Okulicz W.C., McCracken J.A., Schramm W.S. and Robidoux W.F., Jr. Rapid recovery of nuclear estrogen receptor and oxytocin receptor in the ovine uterus following progesterone withdrawal. Journal Steroid Biochemistry 22:686, 1985).

Current hormonal replacement consists of continuous (daily or cyclic) (example day 1-25 of each month) estrogen administration with the addition of a progestin for 10-13 days (example days 13-25) each month. This type of replacement regimen is effective in preventing menopausal symptoms and at the same time, protects the endometrium against the development of hyperplasia or adenocarcinoma. However, the cyclic administration of a progestin leads to a scheduled withdrawal bleed or period in 65-75% of women (see Hellberg D., Nilsson S. Comparison of a triphasic estradiol/norethisterone acetate preparation with and without estriol component in the treatment of climacteric complaints; Maturitas 5:233, 1984; Christensen M.S., Hagen C., Christiansen C., Transbol I. Dose response evaluation of cyclic estrogen/gestagen in postmenopausal women: Placebo controlled trial of its gynecologic and metabolic actions. American Journal of Obstetrics and Gynecology. 144:873, 1982). This withdrawal bleeding is usually not welcomed by the patient and can lead to problems with compliance. Also because the progestin administration is preceded by up to 13-16 days of unopposed estrogen therapy with endometrial proliferation and estrogen and progestin receptor induction, it is possible that a high dose of progestin is required to antagonize these effects resulting in a greater chance of side effects and adverse metabolic effects. Newer continuous low dose estrogen and progestin regimens for hormonal replacement may avoid the problem of withdrawal bleeding (see Magos A.L., Brincatt M., O'Dowd T., et al. Amenorrhea and endometrial atrophy following continuous oral estrogen and progestogen therapy in postmenopausal women. Maturitas 6:145, 1984). However, daily administration of a progestin in these regimens induces depletion of both estrogen and progestin receptors resulting in endometrial atrophy which may be associated with breakthrough bleeding. Since abnormal bleeding in a postmenopausal woman is known to be associated with endometrial carcinoma, it must be investigated by endometrial sampling for hypertrophy usually by D&C. Daily administration of a progestin also raises the concern that the favourable effects of estrogen on HDL cholesterol metabolism will be adversely affected with a fall in HDL cholesterol (see Notelovitz M., Gudat J.C., Ware M.D., Dougherty M.C. British Journal of Obstetrics and Gynecology. 90:171, 1983),
The present invention provides a hormone replacement product containing estrogen and progestin as a combined preparation for administering a plurality of unit dosages of a relatively dominant estrogen activity combination and for administering a plurality of unit dosages of a relatively dominant progestin activity combination in which the unit dosages are arranged for the administration to a female in need of hormone replacement therapy continuously and consecutively alternately of a number of unit dosages of the relatively dominant estrogen activity combination and of a number of unit dosages of the relatively dominant progestin activity combination, each said number of unit dosages consisting of 1 to 5 unit dosages.

Each of said numbers of unit dosages is preferably 2 to 4 and most preferably 3 unit dosages.

The product may be presented in the form of packages each containing 20 to 35 unit dosages.

The female treated by the invention by hormone replacement therapy is for instance of child bearing age or older in whom ovarian estrogen and progesterone production has been interrupted either because of natural menopause, surgical, radiation or chemical ovarian ablation of extirpation or premature ovarian failure.

The invention also provides the new use of estrogen and progestin in the manufacture of the new product.

Thus, in the present disclosure, a formulation is described that is better able to protect the endometrium against the estrogen related risk of endometrial hyperplasia and adenocarcinoma with a lower dose of progestin by administering progestin for a short period of time alternating with a short period of absent or reduced progestin. It has been demonstrated that a protective effect of progestin is related to the duration of administration with 12-13 days per month appearing to be the minimum required for greatest protection. The present formulation administers a low dose of progestin intermittently throughout the month for a minimum of 15 days exposure.

The present invention provides a pharmaceutical preparation for administration to a female of child bearing age or older in whom ovarian estrogen and progesterone production has been interrupted either because of natural menopause, surgical, radiation, or chemical ovarian ablation or extirpation or premature ovarian failure, which comprises a plurality of unit dosages, each unit dosage for continuous consecutive daily administration and comprising combinations of estrogen and progestin selected from a combination with relatively dominant estrogen activity and a combination with relatively dominant progestin activity, with a plurality of dominant estrogen dosages being alternated with plurality of dominant progestin dosages, and each unit dosage also comprising a pharmaceutically acceptable inert carrier when required.

In another aspect, the invention provides the use of estrogen and progestin in a method of manufacture of a product for use in a method of hormonal replacement therapy for administration to a female in need of such treatment, in particular to a female of child bearing age or older to whom ovarian estrogen and progesterone production has been interrupted either because of natural menopause, surgical, radiation, or chemical ovarian ablation or extirpation or premature ovarian failure, which comprises administering continuously and consecutively, in daily sequence, alternately of a number of relatively dominant progestin activity unit dosages and of a number of the dominant estrogen activity unit dosages, each said number of unit dosages consisting of 1 to 5 unit dosages.

The hormonal replacement formulation of the present invention results in the absence of withdrawal bleeding; intermittent increases in estrogen activity; and stimulation of endometrial growth and progestin receptors. This makes the endometrium more sensitive to subsequent progestin activity which limits growth by decreasing estrogen receptors and increasing 17beta-hydroxysteroid dehydrogenase. Interaction of progestin with progestin receptors induces secretory changes in the endometrium which results in a denser stroma and endometrial stability. A return to relatively dominant estrogenic activity then again stimulates estrogen and progestin receptors and renews endometrial sensitivity to progestin. This push/pull activity keeps endometrial activity with a narrow range depending on the number of days of estrogenic and progestagenic activity and maintains a stable endometrium resulting in the absence of breakthrough or withdrawal bleeding.

This hormonal replacement formulation allows better progestational effects with less progestin. With current formulation the dose of progestin is significantly decreased compared with a preparation containing constant daily administration of a progestin. A total steroid dosage is achieved which is similar to or even lower than that of the present cyclic method of administering estrogen and progestin for hormonal replacement therapy of ovarian failure. A reduction in progestin dosage results in less negative impact on HDL cholesterol levels. HDL cholesterol is increased by estrogen and decreased by progestin.

The estrogens which may be employed as a component in the regimens of this invention may be any of those conventionally available. Typically, the estrogen may be selected from the group comprising synthetic and natural estrogens. The synthetic estrogens may be slected from, for example, ethinyl estradiol, mestranol and quinestranol. Particularly of interest are 17alpha-ethinylestradiol and esters and ethers thereof. The preferred estrogen is 17alpha-ethinylestradiol. The natural estrogens may include, for example, conjugated equine estrogens, 17-beta-estradiol, estradiol valerate, estrone, piperazine estrone sulphate, estriol, estriol succinate and polyestrol phosphate.

The progestin component may be any progestationally active compound. Thus, the progestin may be selected from progesterone and its derivatives such as, for example, 17-hydroxy progesterone esters, 19-nor-17-hydroxy progesterone esters, 17alpha-ethinyltestosterone and derivatives thereof, 17alpha-ethinyl-19-nor-testosterone and derivatives thereof, norethindrone, norethindrone acetate, ethynodiol diacetate, dydrogesterone, medroxy-progesterone acetate, norethynodrel, allylestrenol, lynoestrenol, fuingestanol acetate, medrogestone, norgestrienone, dimethiderome, ethisterone, cyproterone, laevo-norgestrel, d-norgestrel, dl-norgestrel, d-17alpha-acetoxy-13beta-ethyl-17alpha-ethinyl-gon-4-en-3-one oxime, cyproterone acetate, gestodene, desogestrel and norgestimate. Preferred progestins are norethindrone, d-norgestrel and norgestimate.

In the invention, the plurality of dosages may comprise from one to five unit dosages, but preferably three unit dosages are employed. Thus, in a preferred form of the invention, three unit dosages of relatively dominant estrogen activity are alternated with three unit dosages of relatively dominant progestin activity.

Generally, the quantities of estrogen and progestin incorporated in the formulation of the invention are dependent on the type of estrogen or progestin selected. However, the quantities employed are generally less than those used in the currently marketed formulations for reasons mentioned earlier. In the present formulation, the estrogen level is kept constant, while the progestin level is adjusted up or down to produce the required estrogen or progestin dominance. The selection of quantity is dependent on the type of estrogen or progestin since each hormone has its own specific activity.

Typically in the hormone replacement formulations, the amount of estrogen per unit dose may range from a minimum of about 0.3 mg of estrone sulphate or its equivalent to a maximum of about 2.5 mg of estrone sulphate or its equivalent. The amount of progestin per unit dosage may range from a minimum of 0 mg to a maximum of about 5 mg of norethindrone or its equivalent. (These amounts generally refer to oral dosages.)

Some preferred combinations include the following:
1. Three units dosages of 0.75 mg piperazine estrone sulphate alternating with three unit dosages of 0.75 mg of piperazine estrone sulphate with 0.35 mg of norethindrone.
2. Three unit dosages of 0.75 mg piperazine estrone sulphate and 0.15 mg of norethindrone alternating with three unit dosages of 0.75 mg of piperazine estrone sulphate and 0.35 mg of norethindrone.

The above combinations may also be grouped into three's and four's, starting with either three or four day groups and ending with the other. For the hormone replacement therapy it may be advantageous for the groups of dominant estrogen activity combination to comprise one more unit dosage than the dominant progestin activity combination unit dosages, or vice versa, for instance the numbers in the groups being 2 and 3 or 3 and 4.

The formulations of the invention may be administered orally, preferably in tablet form, parenterally, sublingually, transdermally, intravaginally, intranasally or buccally. The method of administration determines the types of estrogens and progestins useful in the formulation, as well as the amounts per unit dosage.

Methods for transdermal administration including the associated methods for manufacturing such systems are well known in the art. In this connection, reference may be had to U.S. Patents Nos. 4,752,478, 4,685,911, 4,438,139 and 4,291,014.

Generally speaking, the formulations are prepared according to conventionally known procedures in accordance with the method of administration. Thus, the active ingredients are prepared according to known methods in a pharmaceutically acceptable form for administration. These ingredients, in their required quantities are combined with the appropriate pharmaceutical carriers such as additives, vehicles and/or flavour ameliorating substances. These substances may be referred to as diluents, binders and lubricants. Gums, starches and sugars are also common terms. Typical of these types of substances or excipients are pharmaceutical grades of mannitol, lactose starch, magnesium stearate, sodium saccharin, talcum, cellulose, glucose, sucrose, magnesium carbonate and the like. The active ingredient(s) may comprise from about 0.01% by weight to about 99.99% by weight of the total formulation and the remainder comprises the pharmaceutically acceptable carrier. The percentage of active ingredient(s) may vary according to the delivery system or method of administration and is chosen in accordance with conventional methods known in the art.

Thus, the active ingredients are compounded with the chosen carrier and in for example the case of a tablet form, placed in a tablet molding apparatus to form the tablets which are subsequently packaged in accordance with the chosen regimen.

In the oral form of the formulation, the unit dosages are preferably produced in the form of a pharmaceutical kit or package, with the daily dosages arranged for proper sequential administration. Thus, in another aspect, the present invention also provides a pharmaceutical package which contains combination-type unit dosages in multiple dosage units in a synchronized, fixed sequence, wherein the sequence or arrangement of the dosage units corresponds to the stages of daily administration. This can be known as a multi-preparation pack.

Preferably, such packages are in the form of a transparent package with twenty-eight dosage units arranged sequentially.

Preferably the tablets containing the hormones are different colours or shapes. Data indications may be provided on the packaging. The packaging may be a tube or box or a strip. The box may be circular, square, or otherwise shaped with the tablets being accommodated separately therein for ease of administration. Date indications may appear adjacent each tablet corresponding with the days on which each tablet is to be taken. Some indication of the sequence in which the tablets are to be taken preferably appears on the packaging regardless of its form.

In the following examples, specific embodiments of the present invention are set forth. These are meant to be illustrative of the invention. All parts and percentages are by weight, unless indicated otherwise.

Examples Illustrating the Hormone-Replacement Therapy.

### EXAMPLE 1

Three-day phases of unit dosages of 0.75 mg piperazine estrone sulphate alternating with three-day phases of unit dosages of estrone sulphate 0.75 mg and NET 0.35 mg given continuously and orally.

### EXAMPLE 2

Three-day phases (unit dosages of estrone sulphate 0.75 mg and norethindrone 0.15 mg) alternating with three-day phases of estrone sulphate 0.75 mg and norethindrone 0.35 mg given continuously and orally.

### EXAMPLE 3

Three-day phases of oral micronized 17beta-estradiol 1 mg alternating with three-days of 17beta-estradiol 1 mg and norethindrone .35 mg given continuously.

### EXAMPLE 4

Three-day phases of transdermal 17beta-estradiol (100 ug/day) alternating with three-days of transdermal 17beta-estradiol (100 ug/day) and transdermal norethindrone (.35 mg/day) given continuously.

### EXAMPLE 5

Three-day phases of estrone sulphate 1.25 mg alternating with three-day phases of estrone sulphate 1.25 mg and norethindrone 0.35 mg given continuously and orally.

### EXAMPLE 6

Three-day phases of estrone sulphate 1.25 mg alternating with three-day phases of estrone sulphate 1.25 mg and norethindrone 0.5 mg given continuously and orally.

### EXAMPLE 7

One-day or two-day alternating phases using the unit dosages set forth in Examples 1 and 2 given continuously and orally.

### EXAMPLE 8

Three-day phases of estrone sulphate 0.75 mg alternating with three-day phases of estrone sulphate 0.75 mg and norgestimate 0.050 mg given continuously and orally.

### EXAMPLE 9

Three-day and four-day phases of each of the combinations as set forth in Examples 1 and 2, starting with either a 3- or 4-day phase and given continuously and orally.

### EXAMPLE 10

Two-day and three-day phases of each of the combinations as set forth in Examples 1 and 2, starting with either a 2- or 3-day phase and given continuously and orally.

Our co-pending divisional application is directed to related contraceptive uses of the cyclic dosage regimen described herein and claimed for use in hormone replacement therapy.

## Claims (Claims for the following Contracting State(s): AT, BE, CH, DE, FR, GB, IT, LI, LU, NL, SE)

1. A hormone replacement preparation containing estrogen and progestin as a combined preparation in which a plurality of unit dosages are arranged for the continuous and consecutive administration to a female in need of hormone replacement alternately of a number of relatively dominant estrogen activity unit dosages and of a number of relatively dominant progestin activity unit dosages, each said number of unit dosages consisting of 1 to 5 unit dosages.

2. A preparation according to claim 1 wherein the female to be treated is of child bearing age or older in whom ovarian estrogen and progesterone production has been interrupted either because of natural menopause, surgical, radiation or chemical ovarian ablation or extirpation or premature ovarian failure.

3. A preparation according to claim 1 or 2 in which each said number of unit dosages consists of 2 to 4 unit dosages.

4. A preparation according to any preceding claim in which each said number of unit dosages consists of 3 unit dosages.

5. A preparation according to any preceding claim in which unit dosages are administered orally, parenterally, sublingually, transdermally, intravaginally, intranasally or buccally.

6. A preparation according to claim 5 in which unit dosages are administered orally.

7. A preparation according to claim 5 in which unit dosages are administered transdermally.

8. A preparation according to any preceding claim in which the estrogen is selected from synthetic estrogens, selected from 17alpha-ethinylestradiol and esters and ethers thereof, ethinyl estradiol, mestranol and quinestranol and from natural estrogens selected from conjugated equine estrogens, 17beta-estradiol, estradiol valerate, estrone, estrone sulphate, piperazine estrone sulphate, estriol, estriol succinate and polyestrol phosphate.

9. A preparation according to claim 8 in which the estrogen is slected from estrone sulphate, piperazine estrone sulphate and 17beta estradiol.

10. A preparation according to any preceding claim in which the progestin is selected from progesterone, 17-hydroxy progesterone esters, 19-nor-17-hydroxyprogesterone esters, 17alpha-ethinyl-testosterone, and derivatives thereof 17alpha-ethinyl-19-nor-testosterone and derivatives thereof, norethindrone, norethindrone acetate, ethynodiol diacetate, dydrogesterone, medroxyprogesterone acetate, norethynodrel, allylestrenol, lynoestrenol, quingestanol acetate medrogestone, norgestrienone, dimethisterone, ethisterone, cyproterone, cyproterone acetate, levo-norgestrel, d-norgestrel, dl-norgestrel, d-17alpha-acetoxy-13beta-ethyl-17alpha-ethinyl-gon-4-en-3-one oxime, gestodene, norgestimate and desogestrel.

11. A preparation according to claim 10 in which the progestin is norethindrone or norgestimate or progesterone.

12. A preparation according to any preceding claim in which the amount of estrogen per unit dosage ranges from a minimum of about 0.3 mg to a maximum of about 5.0 mg of piperazine estrone sulphate or its equivalent dosage in another synthetic or natural estrogen and the amount of progestin per unit dosage may range from a minimum of about 0.0 mg to a maximum of about 5.0 mg of norethindrone or its equivalent in a synthetic or natural progestin.

13. A preparation according to claim 12 in which the amount of estrogen per unit dosage ranges from 0.3 mg to 2.5 mg of piperazine estrone sulphate or its equivalent.

14. A preparation according to claim 12 or 13 in which the unit dosages are for oral administration and each said number is 1, 2, 3 or 4 and the relatively dominant estrogen activity combination unit dosages contain 0.75 mg piperazine estrone sulphate and the relatively dominant progestin activity combination unit dosages contain 0.75 mg piperazine estrone sulphate and 0.35 mg norethindrone.

15. A preparation according to claim 12 or 13 in which the unit dosages are for oral administration and each said number is 1, 2, 3 or 4 and in which the dominant estrogen combination unit dosages contain 0.75 mg piperazine estrone sulphate and 0.15 mg norethindrone and the dominant progestin combination unit dosages contain 0.75 piperazine estrone sulphate and 0.35 mg norethindrone.

16. A preparation according to claim 14 or 15 in which the unit dosages are for administration in alternating groups of three of each type of hormone-containing unit dosage.

17. A preparation acording to claim 12 or 13 in which each said number is 3 and in which the relatively dominant estrogen activity combination dosages contain 1 mg 17-beta estradiol and the relatively dominant progestin activity combination dosages contain 1 mg 17-beta estradiol and 0.35 mg norethindrone and each is administered orally.

18. A preparation according to claim 12 or 13 in which each said number is 3 and in which the relatively dominant estrogen activity combination unit dosage contains 0.75 mg estrone sulphate and the relatively dominant progestin activity combination unit dosage contains 0.75 mg estrone sulphate and 0.050 mg norgestimate each administered orally.

19. A preparation according to claim 12 or 13 in which each said number is 3 and in which the relatively dominant estrogen activity combination unit dosage contains 1.25 mg estrone sulphate and the relatively dominant progestin combination contains 1.25 mg estrone sulphate and either 0.35 mg or 0.5 mg norethindrone each administered orally.

20. A preparation according to claim 12 or 13 in which each said number is 1, 2, 3 or 4 and in which the relatively dominant estrogen activity combination contains 0.75 mg estrone sulphate and 0.15 mg norethindrone and the relatively dominant progestin activity combination contains 0.75 estrone sulphate and 0.35 mg norethindrone each administered orally.

21. A preparation according to claim 12 or 13 in which the unit dosages are for transdermal administration and are for administration in alternating three day periods of 17beta-estradiol at 0.1 mg/day and three day periods of 17beta-estradiol at 0.1 mg/day plus norethindrone at 0.35 mg/day.

22. Use of estrogen and progestin in a method of manufacturing a product characterised in that the product is for hormone replacement therapy and contains estrogen and progestin as a combined preparation for administering to a female in need of hormone therapy continuously and consecutively alternately of a number of relatively dominant estrogen activity unit dosages and of a number of relatively dominant progestin activity unit dosages, each said number of unit dosages consisting of 1 to 5 unit dosages.

23. Use according to claim 22 which is for administration to a female of child bearing age or older in whom ovarian estrogen and progesterone production has been interrupted either because of natural menopause, surgical, radiation or chemical ovarian ablation or extirpation or premature ovarian failure.

24. Use according to claim 22 or 23 in which the product has the further features as defined in any of claims 3 to 21.

## Claims (Claims for the following Contracting State(s): ES, GR)

1. Use of estrogen and progestin in a process of manufacturing a product characterised in that the product is for hormone replacement therapy and contains estrogen and progestin as a combined preparation for administering to a female in need of hormone therapy continuously and consecutively alternately of a number of relatively dominant estrogen activity unit dosages and of a number of relatively dominant progestin activity unit dosages, each said number of unit dosages consisting of 1 to 5 unit dosages.

2. Use according to claim 1 wherein the female to be treated is of child bearing age or older in whom ovarian estrogen and progesterone production has been interrupted either because of natural menopause, surgical, radiation or chemical ovarian ablation or extirpation or premature ovarian failure.

3. Use according to claim 1 or 2 in which each said number of unit dosages consists of 2 to 4 unit dosages.

4. Use according to any preceding claim in which each said number of unit dosages consists of 3 unit dosages.

5. Use according to any preceding claim in which unit dosages are suitable for administration orally, parenterally, sublingually, transdermally, intravaginally, intranasally or buccally.

6. Use according to claim 5 in which unit dosages are suitable for administration orally.

7. Use according to claim 5 in which unit dosages are suitable for administration transdermally.

8. Use according to any preceding claim in which the estrogen is selected from synthetic estrogens, selected from 17alpha-ethinylestradiol and esters and ethers thereof, ethinyl estradiol, mestranol and quinestranol and from natural estrogens selected from conjugated equine estrogens, 17beta-estradiol, estradiol valerate, estrone, estrone sulphate, piperazine estrone sulphate, estriol, estriol succinate and polyestrol phosphate.

9. Use according to claim 8 in which the estrogen is slected from estrone sulphate, piperazine estrone sulphate and 17beta estradiol.

10. Use according to any preceding claim in which the progestin is selected from progesterone, 17-hydroxy progesterone esters, 19-nor-17-hydroxy- progesterone esters, 17alpha-ethinyl-testosterone, and derivatives thereof 17alpha-ethinyl-19-nor-testosterone and derivatives thereof, norethindrone, norethindrone acetate, ethynodiol diacetate, dydrogesterone, medroxyprogesterone acetate, norethynodrel, allylestrenol, lynoestrenol, quingestanol acetate medrogestone, norgestrienone, dimethisterone, ethisterone, cyproterone, cyproterone acetate, levonorgestrel, d-norgestrel, dl-norgestrel, d-17alpha-acetoxy-13beta-ethyl-17alpha-ethinyl-gon-4-en-3-one oxime, gestodene, norgestimate and desogestrel.

11. Use according to claim 10 in which the progestin is norethindrone or norgestimate or progesterone.

12. Use according to any preceding claim in which the amount of estrogen per unit dosage ranges from a minimum of about 0.3 mg to a maximum of about 5.0 mg of piperazine estrone sulphate or its equivalent dosage in another synthetic or natural estrogen and the amount of progestin per unit dosage may range from a minimum of about 0.0 mg to a maximum of about 5.0 mg of norethindrone or its equivalent in a synthetic or natural progestin.

13. Use according to claim 12 in which the amount of estrogen per unit dosage ranges from 0.3 mg to 2.5 mg of piperazine estrone sulphate or its equivalent.

14. Use according to claim 12 or 13 in which the unit dosages are for oral administration and each said number is 1, 2, 3 or 4 and the relatively dominant estrogen activity combination unit dosages contain 0.75 mg piperazine estrone sulphate and the relatively dominant progestin activity combination unit dosages contain 0.75 mg piperazine estrone sulphate and 0.35 mg norethindrone.

15. Use according to claim 12 or 13 in which the unit dosages are for oral administration and each said number is 1, 2, 3 or 4 and in which the dominant estrogen combination unit dosages contain 0.75 mg piperazine estrone sulphate and 0.15 mg norethindrone and the dominant progestin combination unit dosages contain 0.75 piperazine estrone sulphate and 0.35 mg norethindrone.

16. Use according to claim 14 or 15 in which the unit dosages are for administration in alternating groups of three of each type of hormone-containing unit dosage.

17. Use according to claim 12 or 13 in which each said number is 3 and in which the relatively dominant estrogen activity combination dosages contain 1 mg 17-beta estradiol and the relatively dominant progestin activity combination dosages contain 1 mg 17-beta estradiol and 0.35 mg norethindrone and each is administered orally.

18. Use according to claim 12 or 13 in which each said number is 3 and in which the relatively dominant estrogen activity combination unit dosage contains 0.75 mg estrone sulphate and the relatively dominant progestin activity combination unit dosage contains 0.75 mg estrone sulphate and 0.050 mg norgestimate each administered orally.

19. Use according to claim 12 or 13 in which each said number is 3 and in which the relatively dominant estrogen activity combination unit dosage contains 1.25 mg estrone sulphate and the relatively dominant progestin combination contains 1.25 mg estrone sulphate and either 0.35 mg or 0.5 mg norethindrone each administered orally.

20. Use according to claim 12 or 13 in which each said number is 1, 2, 3 or 4 and in which the relatively dominant estrogen activity combination contains 0.75 mg estrone sulphate and 0.15 mg norethindrone and the relatively dominant progestin activity combination contains 0.75 estrone sulphate and 0.35 mg norethindrone each administered orally.

21. Use according to claim 12 or 13 in which the unit dosages are for transdermal administration and are for administration in alternating three day periods of 17beta-estradiol at 0.1 mg/day and three day periods of 17beta-estradiol at 0.1 mg/day plus norethindrone at 0.35 mg/day.

22. A process of manufacturing a product characterised in that the product is for hormone replacement therapy and contains estrogen and progestin as a combined preparation for administering to a female in need of hormone therapy continuously and consecutively alternately of a number of relatively dominant estrogen activity unit dosages and of a number of relatively dominant progestin activity unit dosages, each said number of unit dosages consisting of 1 to 5 unit dosages.

23. A process of manufacturing a hormone replacement product comprising estrogen and progestin, comprising the steps of selecting an amount of estrogen and an amount of progestin suitable for providing a relatively dominant estrogen activity unit dosage, mixing each of the estrogen and progestin of the relatively dominant estrogen activity dosage with a pharmceutical carrier, selecting an amount of estrogen and progestin suitable for providing a relatively dominant progestin activity unit dosage, mixing each of the estrogen and progestin of the relatively dominant progestin activity dosage with a pharmaceutical carrier, and packaging the unit dosages in an arrangement for administering continuously and consecutively alternately of a number of relatively dominant estrogen activity unit dosages and of a number of relatively dominant progestin activity unit dosages, each said number of unit dosages consisting of 1 to 5 unit dosages.

24. A process according to claim 23 in which the unit dosages are molded to form tablets and the tablets are packaged in a transparent package each comprising twenty-eight dosage units arranged in a fixed sequence.

25. A process according to claim 24 in which the package includes date indications adjacent each tablet each indicating the day on which the corresponding tablet is to be taken.

26. A process according to any of claims 22 to 25 in which the product is for administration to a female who is of child bearing age or older in whom ovarian estrogen and progesterone production has been interrupted either because of natural menopause, surgical, radiation or chemical ovarian ablation or extirpation or premature ovarian failure.

27. A process according to any of claims 22 to 26 which has the further features defined in any of claims 3 to 21.

## Patentansprüche (Patentansprüche für folgende(n) Vertragsstaat(en): AT, BE, CH, DE, FR, GB, IT, LI, LU, NL, SE)

1. Präparat zum Hormonersatz, das Östrogen und Progestin als ein Kombinationspräparat enthält, bei dem eine Menge Dosierungseinheiten kontinuierlich und aufeinanderfolgend angeordnet sind zur Verabreichung abwechselnd einer Anzahl Dosierungseinheiten mit relativ dominanter Östrogenaktivität und einer Anzahl Dosierungseinheiten mit relativ dominanter Progestinaktivität an eine Frau, die Hormonersatz benötigt, wobei eine jede Anzahl Dosierungseinheiten aus 1 bis 5 Dosierungseinheiten besteht.

2. Präparat nach Anspruch 1, wobei eine Frau im gebärfähigen Alter oder älter behandelt wird, bei der die Produktion von Östrogen und Progesteron in den Ovarien entweder aufgrund der natürlichen Menopause, wegen chirurgischer, bestrahlungsbedingter oder chemischer Entfernung oder Extirpation der Eierstöcke oder wegen verfrühten Versagens der Eierstöcke unterbrochen ist.

3. Präparat nach Anspruch 1 oder 2, bei dem eine jede Anzahl Dosierungseinheiten aus 2 bis 4 Dosierungseinheiten besteht.

4. Präparat nach irgendeinem der vorhergehenden Ansprüche, bei dem eine jede Anzahl Dosierungseinheiten aus 3 Dosierungseinheiten besteht.

5. Präparat nach irgendeinem der vorhergehenden Ansprüche, bei dem die Dosierungseinheiten oral, parenteral, sublingual, transdermal, intravaginal, intranasal oder bukkal verabreicht werden.

6. Präparat nach Anspruch 5, bei dem die Dosierungseinheiten oral verabreicht werden.

7. Präparat nach Anspruch 5, bei dem die Dosierungseinheiten transdermal verabreicht werden.

8. Präparat nach irgendeinem der vorhergehenden Ansprüche, bei dem das Östrogen ausgewählt ist aus synthetischen Östrogenen, ausgewählt aus 17α-Ethinylestradiol und dessen Estern und Ethern, Ethinylestradiol, Mestranol und Chinestranol, und aus natürlichen Östrogenen, ausgewählt aus konjugierten Pferdeestrogenen, 17β-Östradiol, Östradiolvalerianat, Östron, Östronsulfat, Piperazin-estronsulfat, Östriol, Östriolsuccinat und Polyestrolphosphat.

9. Präparat nach Anspruch 8, bei dem das Östrogen ausgewählt ist aus Östronsulfat, Piperazin-estronsulfat und 17β-Östradiol.

10. Präparat nach irgendeinem der vorhergehenden Ansprüche, bei dem das Progestin ausgewählt ist aus Progesteron, 17-Hydroxyprogesteronestern, 19-Nor-17-Hydroxyprogesteronestern, 17α-Ethinyltestosteron und dessen Derivaten, 17α-Ethinyl-19-nortestosteron und dessen Derivaten, Norethisteron, Norethisteronacetat, Ethynodioldiacetat, Dydrogesteron, Medroxyprogesteronacetat, Norethynodrel, Allylestrenol, Lynoestrenol, Chingestanolacetat, Medrogeston, Norgestrienon, Dimethisteron, Ethisteron, Cyproteron, Cyproteronacetat, Lävo-Norgestrel, d-Norgestrel, dl-Norgestrel, d-17α-Acetoxy-13β-ethyl-17α-ethinyl-gon-4-en-3-on-oxim, Gestoden, Norgestimat und Desogestrel.

11. Präparat nach Anspruch 10, bei dem das Progestin Norethisteron oder Norgestimat oder Progesteron ist.

12. Präparat nach irgendeinem der vorhergehenden Ansprüche, bei dem die Menge Östrogen pro Dosierungseinheit zwischen einem Minimum von ungefähr 0,3 mg und einem Maximum von ungefähr 5,0 mg Piperazin-estronsulfat oder dessen Äquivalentdosierung eines anderen synthetischen oder natürlichen Östrogens liegt, und die Menge Progestin pro Dosierungseinheit zwischen einem Minimum von ungefähr 0,0 mg und einem Maximum von ungefähr 5,0 mg Norethisteron oder dessen Äquivalent eines synthetischen oder natürlichen Progestins liegen kann.

13. Präparat nach Anspruch 12, bei dem die Menge Östrogen pro Dosierungseinheit zwischen 0,3 mg und 2,5 mg Piperazinestronsulfat oder dessen Äquivalent liegt.

14. Präparat nach Anspruch 12 oder 13, bei dem die Dosierungseinheiten zur oralen Verabreichung bestimmt sind und eine jede Anzahl 1, 2, 3, oder 4 ist und die Dosierungseinheiten mit einer Kombination mit relativ dominanter Östrogenaktivität 0,75 mg Piperazin-estronsulfat enthalten und die Dosierungseinheiten mit einer Kombination relativ dominanter Progestinaktivität 0,75 mg Piperazin-estronsulfat und 0,35 mg Norethisteron enthalten.

15. Präparat nach Anspruch 12 oder 13, bei dem die Dosierungseinheiten zur oralen Verabreichung bestimmt sind und eine jede Anzahl 1, 2, 3, oder 4 ist und bei der die Dosierungseinheiten mit einer Kombination mit dominanter Östrogenaktivität 0,75 mg Piperazin-estronsulfat und 0,15 mg Norethisteron enthalten und die Dosierungseinheiten mit einer Kombination dominanter Progestinaktivität 0,75 mg Piperazin-estronsulfat und 0,35 mg Norethisteron enthalten.

16. Präparat nach Anspruch 14 oder 15, bei dem die Dosierungseinheiten jeder Art der hormonhaltigen Dosierungseinheit zur Verabreichung in alternierenden Dreiergruppen vorliegen.

17. Präparat nach Anspruch 12 oder 13, bei dem eine jede Anzahl 3 ist, und bei dem die Dosierung mit einer Kombination mit relativ dominanter Östrogenaktivität 1 mg 17β-Östradiol enthalten und die Dosierung mit einer Kombination relativ dominanter Progestinaktivität 1 mg 17β-Östradiol und 0,35 mg Norethisteron enthalten und jeweils oral verabreicht werden.

18. Präparat nach Anspruch 12 oder 13, bei dem eine jede Anzahl 3 ist, und bei dem die Dosierungseinheit mit einer Kombination mit relativ dominanter Östrogenaktivität 0,75 mg Östronsulfat enthält und die Dosierungseinheit mit einer Kombination mit relativ dominanter Progestinaktivität 0,75 mg Östronsulfat und 0,050 mg Norgestimat enthält, jeweils oral verabreicht.

19. Präparat nach Anspruch 12 oder 13, bei dem eine jede Anzahl 3 ist, und bei dem die Dosierungseinheit mit einer Kombination mit relativ dominanter Östrogenaktivität 1,25 mg Östronsulfat enthält und die Kombination mit relativ dominanter Progestinaktivität 1,25 mg Östronsulfat und entweder 0,35 mg oder 0,5 mg Norethisteron enthält, jeweils oral verabreicht.

20. Präparat nach Anspruch 12 oder 13, bei dem eine jede Anzahl 1, 2, 3 oder 4 ist, und bei dem die Kombination mit relativ dominanter Östrogenaktivität 0,75 mg Östronsulfat und 0,15 mg Norethisteron enthält und die Kombination mit relativ dominanter Progestinaktivität 0,75 mg Östronsulfat und 0,35 mg Norethisteron enthält, jeweils oral verabreicht.

21. Präparat nach Anspruch 12 oder 13, bei dem die Dosierungseinheiten zur transdermalen Verabreichung und zur Verabreichung in alternierenden Dreitagesphasen mit 0,1 mg/Tag 17β-Östradiol und Dreitagesphasen mit 0,1 mg/Tag 17β-Östradiol plus 0,1 mg/Tag Norethisteron bestimmt sind.

22. Verwendung von Östrogen und Progestin in einem Verfahren zur Herstellung eines Erzeugnisses, dadurch gekennzeichnet, daß das Erzeugnis zur Hormonersatztherapie bestimmt ist und Östrogen und Progestin als Kombinationspräparat enthält, zur kontinuierlichen und aufeinanderfolgenden Verabreichung abwechselnd einer Anzahl Dosierungseinheiten mit relativ dominanter Östrogenaktivität und einer Anzahl von Dosierungseinheiten mit relativ dominanter Progestinaktivität an eine Frau, die Hormonersatz benötigt, wobei eine jede Anzahl Dosierungseinheiten aus 1 bis 5 Dosierungseinheiten besteht.

23. Verwendung nach Anspruch 22 zur Verabreichung an eine Frau im gebärfähigen Alter oder älter, bei der die Produktion an Östrogen und Progesteron in den Ovarien entweder aufgrund der natürlichen Menopause, wegen chirurgischer, bestrahlungsbedingter oder chemischer Entfernung oder Extirpation der Eierstöcke oder wegen verfrühten Versagens der Eierstöcke unterbrochen ist.

24. Verwendung nach Anspruch 22 oder 23, bei der das Erzeugnis die weiteren in irgendeinem der Ansprüche 3 bis 21 definierten Kennzeichen hat.

## Patentansprüche (Patentansprüche für folgende(n) Vertragsstaat(en): ES, GR)

1. Verwendung von Östrogen und Progestin in einem Verfahren zur Herstellung eines Erzeugnisses, dadurch gekennzeichnet, daß das Erzeugnis zur Hormonersatztherapie bestimmt ist und Östrogen und Progestin als Kombinationspräparat enthält, zur kontinuierlichen und aufeinanderfolgenden Verabreichung abwechselnd einer Anzahl Dosierungseinheiten mit relativ dominanter Östrogenaktivität und einer Anzahl Dosierungseinheiten mit relativ dominanter Progestinaktivität an eine Frau, die Hormonersatz benötigt, wobei eine jede Anzahl Dosierungseinheiten aus 1 bis 5 Dosierungseinheiten besteht.

2. Verwendung nach Anspruch 1, wobei die zu behandelnde Frau eine Frau im gebärfähigen Alter oder älter ist, bei der die Produktion von Östrogen und Progesteron in den Ovarien entweder aufgrund der natürlichen Menopause, wegen chirurgischer, bestrahlungsbedingter oder chemischer Entfernung oder Extirpation der Eierstöcke oder wegen verfrühten Versagens der Eierstöcke unterbrochen ist.

3. Verwendung nach Anspruch 1 oder 2, bei der eine jede Anzahl Dosierungseinheiten aus 2 bis 4 Dosierungseinheiten besteht.

4. Verwendung nach irgendeinem der vorhergehenden Ansprüche, bei der eine jede Anzahl Dosierungseinheiten aus 3 Dosierungseinheiten besteht.

5. Verwendung nach irgendeinem der vorhergehenden Ansprüche, bei der die Dosierungseinheiten zur oralen, parenteralen, sublingualen, transdermalen, intravaginalen, intranasalen oder bukkalen Verabreichung geeignet sind.

6. Verwendung nach Anspruch 5, bei der die Dosierungseinheiten zur oralen Verabreichung geeignet sind.

7. Verwendung nach Anspruch 5, bei der die Dosierungseinheiten zur transdermalen Verabreichung geeignet sind.

8. Verwendung nach irgendeinem der vorhergehenden Ansprüche, bei der das Östrogen ausgewählt ist aus synthetischen Östrogenen, ausgewählt aus 17α-Ethinylestradiol und dessen Estern und Ethern, Ethinylestradiol, Mestranol und Chinestranol, und aus natürlichen Östrogenen, ausgewählt aus konjugierten Pferdeestrogenen, 17β-Östradiol, Östradiolvalerianat, Östron, Östronsulfat, Piperazin-estronsulfat, Östriol, Östriolsuccinat und Polyestrolphosphat.

9. Verwendung nach Anspruch 8, bei der das Östrogen ausgewählt ist aus Östronsulfat, Piperazin-estronsulfat und 17β-Östradiol.

10. Verwendung nach irgendeinem der vorhergehenden Ansprüche, bei der das Progestin ausgewählt ist aus Progesteron, 17-Hydroxyprogesteronestern, 19-Nor-17-Hydroxyprogesteronestern, 17α-Ethinyltestosteron und dessen Derivaten, 17α-Ethinyl-19-nortestosteron und dessen Derivaten, Norethisteron, Norethisteronacetat, Ethynodioldiacetat, Dydrogesteron, Medroxyprogesteronacetat, Norethynodrel, Allylestrenol, Lynoestrenol, Chingestanolacetat, Medrogeston, Norgestrienon, Dimethisteron, Ethisteron, Cyproteron, Cyproteronacetat, Lävo-Norgestrel, d-Norgestrel, dl-Norgestrel, d-17α-Acetoxy-13β-ethyl-17α-ethinyl-gon-4-en-3-on-oxim, Gestoden, Norgestimat und Desogestrel.

11. Verwendung nach Anspruch 10, bei der das Progestin Norethisteron oder Norgestimat oder Progesteron ist.

12. Verwendung nach irgendeinem der vorhergehenden Ansprüche, bei der die Menge Östrogen pro Dosierungseinheit zwischen einem Minimum von ungefähr 0,3 mg und einem Maximum von ungefähr 5,0 mg Piperazin-estronsulfat oder dessen Äquivalentdosierung eines anderen synthetischen oder natürlichen Östrogens liegt, und die Menge Progestin pro Dosierungseinheit zwischen einem Minimum von ungefähr 0,0 mg und einem Maximum von ungefähr 5,0 mg Norethisteron oder dessen Äquivalent eines synthetischen oder natürlichen Progestins liegen kann.

13. Verwendung nach Anspruch 12, bei der die Menge Östrogen pro Dosierungseinheit zwischen 0,3 mg und 2,5 mg Piperazin-estronsulfat oder dessen Äquivalent liegt.

14. Verwendung nach Anspruch 12 oder 13, bei der die Dosierungseinheiten zur oralen Verabreichung bestimmt sind und eine jede Anzahl 1, 2, 3, oder 4 ist und die Dosierungseinheiten mit einer Kombination mit relativ dominanter Östrogenaktivität 0,75 mg Piperazin-estronsulfat enthalten und die Dosierungseinheiten mit einer Kombination mit relativ dominanter Progestinaktivität 0,75 mg Piperazin-estronsulfat und 0,35 mg Norethisteron enthalten.

15. Verwendung nach Anspruch 12 oder 13, bei der die Dosierungseinheiten zur oralen Verabreichung bestimmt sind und eine jede Anzahl 1, 2, 3, oder 4 ist und bei der die Dosierungseinheiten mit einer Kombination mit dominanter Östrogenaktivität 0,75 mg Piperazin-estronsulfat und 0,15 mg Norethisteron enthalten und die Dosierungseinheiten mit einer Kombination dominanter Progestinaktivität 0,75 mg Piperazin-estronsulfat und 0,35 mg Norethisteron enthalten.

16. Verwendung nach Anspruch 14 oder 15, bei der die Dosierungseinheiten jeder Art hormonhaltiger Dosierungseinheit zur Verabreichung in alternierenden Dreiergruppen vorliegen.

17. Verwendung nach Anspruch 12 oder 13, bei der eine jede Anzahl 3 ist, und bei der die Dosierung mit einer Kombination relativ dominanter Östrogenaktivität 1 mg 17β-Östradiol enthalten und die Dosierung mit einer Kombination relativ dominanter Progestinaktivität 1 mg 17β-Östradiol und 0,35 mg Norethisteron enthalten und jeweils oral verabreicht werden.

18. Verwendung nach Anspruch 12 oder 13, bei der eine jede Anzahl 3 ist, und bei der die Dosierungseinheit mit einer Kombination mit relativ dominanter Östrogenaktivität 0,75 mg Östronsulfat enthält und die Dosierungseinheit mit einer Kombination mitrelativ dominanter Progestinaktivität 0,75 mg Östronsulfat und 0,050 mg Norgestimat enthält, jeweils oral verabreicht.

19. Verwendung nach Anspruch 12 oder 13, bei der eine jede Anzahl 3 ist, und bei der die Dosierungseinheit mit einer Kombination mit relativ dominanter Östrogenaktivität 1,25 mg Östronsulfat enthält und die Kombination mit relativ dominanter Progestinaktivität 1,25 mg Östronsulfat und entweder 0,35 mg oder 0,5 mg Norethisteron enthält, jeweils oral verabreicht.

20. Verwendung nach Anspruch 12 oder 13, bei der eine jede Anzahl 1, 2, 3 oder 4 ist, und bei der die Kombination mit relativ dominanter Östrogenaktivität 0,75 mg Östronsulfat und 0,15 mg Norethisteron enthält und die Kombination mit relativ dominanter Progestinaktivität 0,75 mg Östronsulfat und 0,35 mg Norethisteron enthält, jeweils oral verabreicht.

21. Verwendung nach Anspruch 12 oder 13, bei der die Dosierungseinheiten zur transdermalen Verabreichung in alternierenden Dreitagesphasen mit 0,1 mg/Tag 17β-Östradiol und Dreitagesphasen mit 0,1 mg/Tag 17β-Östradiol plus 0,1 mg/Tag Norethisteron bestimmt sind.

22. Verfahren zur Herstellung eines Erzeugnisses, dadurch gekennzeichnet, daß das Erzeugnis zur Hormonersatztherapie bestimmt ist und Östrogen und Progestin als Kombinationspräparat enthält, zur kontinuierlichen und aufeinanderfolgenden Verabreichung abwechselnd einer Anzahl Dosierungseinheiten mit relativ dominanter Östrogenaktivität und einer Anzahl Dosierungseinheiten mit relativ dominanter Progestinaktivität an eine Frau, die Hormonersatz benötigt, wobei eine jede Anzahl Dosierungseinheiten aus 1 bis 5 Dosierungseinheiten besteht.

23. Verfahren zur Herstellung eines Erzeugnisses zum Hormonersatz, das Östrogen und Progestin umfaßt, das die Schritte umfaßt: Auswählen einer Menge Östrogen und einer Menge Progestin, die geeignet sind, eine Dosierungseinheit mit relativ dominanter Östrogenaktivität zu liefern, Mischen des Östrogens und des Progestins der Dosierungseinheit mit relativ dominanter Östrogenaktivität jeweils mit einem pharmazeutischen Träger, Auswählen einer Menge Östrogen und Progestin, die geeignet sind, eine Dosierungseinheit mit relativ dominanter Progestinaktivität zu liefern, Mischen des Östrogens und des Progestins der Dosierungseinheit mit relativ dominanter Progestinaktivität jeweils mit einem pharmazeutischen Träger, und Verpacken der Dosierungseinheiten in einer Anordnung zur kontinuierlichen und aufeinanderfolgenden Verabreichung abwechselnd einer Anzahl Dosierungseinheiten mit relativ dominanter Östrogenaktivität und einer Anzahl Dosierungseinheiten mit relativ dominanter Progestinaktivität, wobei eine jede Anzahl Dosierungseinheiten aus 1 bis 5 Dosierungseinheiten besteht.

24. Verfahren nach Anspruch 23, bei dem die Dosierungseinheiten zu Tabletten geformt werden und die Tabletten in eine transparente Verpackung gepackt werden, wobei jede Packung 28 Dosierungseinheiten umfaßt, die in einer bestimmten Folge angeordnet sind.

25. Verfahren nach Anspruch 24, bei dem die Packung Datumsangaben neben jeder Tablette besitzt, die jeweils den Tag angeben, an dem die jeweilige Tablette einzunehmen ist.

26. Verfahren nach irgendeinem der Ansprüche 22 bis 25, bei dem das Erzeugnis zur Verabreichung an eine Frau im gebärfähigen Alter oder älter bestimmt ist, bei der die Produktion von Östrogen und Progesteron in den Ovarien entweder aufgrund der natürlichen Menopause, wegen chirurgischer, bestrahlungsbedingter oder chemischer Entfernung oder Extirpation der Eierstöcke oder wegen verfrühten Versagens der Eierstöcke unterbrochen ist.

27. Verfahren nach irgendeinem der Ansprüche 22 bis 26, das die weiteren in irgendeinem der Ansprüche 3 bis 21 definierten Kennzeichen hat.

## Revendications (Revendications pour l'(les) Etat(s) contractant(s) suivant(s): AT, BE, CH, DE, FR, GB, IT, LI, LU, NL, SE)

1. Préparation hormonale substitutive contenant un oestrogène et un progestatif sous la forme d'une préparation associée dans laquelle une pluralité de doses unitaires sont conçues pour l'administration continue et consécutive chez une femme nécessitant une substitution hormonale, par alternance, d'un certain nombre de doses unitaires à activité oestrogénique relativement dominante et d'un certain nombre de doses unitaires à activité progestative relativement dominante, chacun desdits nombres de doses unitaires consistant en 1 à 5 doses unitaires.

2. Préparation selon la revendication 1, dans laquelle la femme à traiter est en âge de procréer ou plus âgée et sa production ovarienne d'oestrogène et de progestérone a été interrompue soit à la suite d'une ménopause naturelle, soit à la suite d'une ablation ou d'une extirpation ovarienne chirurgicale, par rayons ou produits chimiques, soit à la suite d'une insuffisance ovarienne précoce.

3. Préparation selon la revendication 1 ou 2, dans laquelle chacun desdits nombres de doses unitaires consiste en 2 à 4 doses unitaires.

4. Préparation selon l'une quelconque des revendications précédentes, dans laquelle chacun desdits nombres de doses unitaires consiste en 3 doses unitaires.

5. Préparation selon l'une quelconque des revendications précédentes, dans laquelle les doses unitaires sont administrées par voie orale, parentérale, sublinguale, percutanée, intravaginale, intranasale ou buccale.

6. Préparation selon la revendication 5, dans laquelle les doses unitaires sont administrées par voie orale.

7. Préparation selon la revendication 5, dans laquelle les doses unitaires sont administrées par voie percutanée.

8. Préparation selon l'une quelconque des revendications précédentes, dans laquelle l'oestrogène est sélectionné parmi les oestrogènes de synthèse sélectionnés parmi le 17α-éthinylestradiol et ses esters et éthers, l'éthinylestradiol, le mestranol et le quinestranol et parmi les oestrogènes naturels sélectionnés parmi les oestrogènes équins conjugués, le 17β-estradiol, le valérate d'estradiol, l'estrone, le sulfate d'estrone, le sulfate d'estrone-pipérazine, l'estriol, le succinate d'estriol et le phosphate de polyestrol.

9. Préparation selon la revendication 8, dans laquelle l'oestrogène est sélectionné parmi le sulfate d'estrone, le sulfate d'estrone-pipérazine et le 17β-estradiol.

10. Préparation selon l'une des revendications précédentes, dans laquelle le progestatif est sélectionné parmi la progestérone, les esters de 17-hydroxyprogestérone, les esters de 19-nor-17-hydroxyprogestérone, la 17α-éthinyltestostérone et ses dérivés, la 17α-éthinyl-19-nortestostérone et ses dérivés, la noréthistérone, l'acétate de noréthistérone, le diacétate d'éthynodiol, la dydrogestérone, l'acétate de médroxyprogestérone, le noréthynodrel, l'allylestrénol, le lynestrénol, l'acétate de quingestanol, la médrogestone, la norgestriénone, la diméthistérone, l'éthistérone, la cyprotérone, l'acétate de cyprotérone, le lévo-norgestrel, le d-norgestrel, le dl-norgestrel, le d-17α-acétoxy-13β-éthyl-17α-éthinylgon-4-ène-3-one oxime, le gestodène, le norgestimate et le désogestrel.

11. Préparation selon la revendication 10, dans laquelle le progestatif est la noréthistérone ou le norgestimate ou la progestérone.

12. Préparation selon l'une des revendications précédentes, dans laquelle la quantité d'oestrogène par dose unitaire est comprise entre un minimum d'environ 0,3 mg et un maximum d'environ 5,0 mg de sulfate d'estrone-pipérazine ou une dose équivalente d'un autre oestrogène synthétique ou naturel et la quantité de progestatif par dose unitaire peut être comprise entre un minimum d'environ 0,0 mg et un maximum d'environ 5,0 mg de noréthistérone ou de son équivalent en progestatif synthétique ou naturel.

13. Préparation selon la revendication 12, dans laquelle la quantité d'oestrogène par dose unitaire est comprise entre 0,3 mg et 2,5 mg de sulfate d'estrone-pipérazine ou son équivalent.

14. Préparation selon la revendication 12 ou 13, dans laquelle les doses unitaires sont destinées à l'administration orale et chacun desdits nombres est 1, 2, 3 ou 4, et les doses unitaires de l'association à activité oestrogénique relativement dominante contiennent 0,75 mg de sulfate d'estrone-pipérazine et les doses unitaires de l'association à activité progestative relativement dominante contiennent 0,75 mg de sulfate d'estrone-pipérazine et 0,35 mg de noréthistérone.

15. Préparation selon la revendication 12 ou 13, dans laquelle les doses unitaires sont destinées à l'administration orale et chacun desdits nombres est 1, 2, 3 ou 4 et dans laquelle les doses unitaires de l'association à dominance oestrogénique contiennent 0,75 mg de sulfate d'estrone-pipérazine et 0,15 mg de noréthistérone et les doses unitaires de l'association à dominance progestative contiennent 0,75 mg de sulfate d'estrone-pipérazine et 0,35 mg de noréthistérone.

16. Préparation selon la revendication 14 ou 15, dans laquelle les doses unitaires sont destinées à l'administration en groupes alternants de 3 pour chaque types de dose unitaire contenant une hormone.

17. Préparation selon la revendication 12 ou 13, dans laquelle chacun desdits nombres est 3 et dans laquelle les doses de l'association à activité oestrogénique relativement dominante contiennent 1 mg de 17β-estradiol et les doses de l'association à activité progestative relativement dominante contiennent 1 mg de 17β-estradiol et 0,35 mg de noréthistérone, l'une et l'autre étant administrées par voie orale.

18. Préparation selon la revendication 12 ou 13, dans laquelle chacun desdits nombres est 3 et dans laquelle la dose unitaire de l'association à activité oestrogénique relativement dominante contient 0,75 mg de sulfate d'estrone et la dose unitaire de l'association à activité progestative relativement dominante contient 0,75 mg de sulfate d'estrone et 0,050 mg de norgestimate, l'une et l'autre administrées par voie orale.

19. Préparation selon la revendication 12 ou 13, dans laquelle chacun desdits nombres est 3 et dans laquelle la dose unitaire de l'association à activité oestrogénique relativement dominante contient 1,25 mg de sulfate d'estrone et l'association à dominance progestative relative contient 1,25 mg de sulfate d'estrone et soit 0,35 mg, soit 0,5 mg de noréthistérone, l'une et l'autre administrées par voie orale.

20. Préparation selon la revendication 12 ou 13, dans laquelle chacun desdits nombres est 1, 2, 3 ou 4 et dans laquelle l'association à activité oestrogénique relativement dominante contient 0,75 mg de sulfate d'estrone et 0,15 mg de noréthistérone et l'association à activité progestative relativement dominante contient 0,75 mg de sulfate d'estrone et 0,35 mg de noréthistérone, l'une et l'autre administrées par voie orale.

21. Préparation selon la revendication 12 ou 13, dans laquelle les doses unitaires sont destinées à l'administration percutanée et à l'administration en alternance par périodes de 3 jours de 17β-estradiol à raison de 0,1 mg/jour et par périodes de 3 jours de 17β-estradiol à raison de 0,1 mg/jour et de noréthistérone à raison de 0,35 mg/jour.

22. Emploi d'un oestrogène et d'un progestatif dans un procédé de préparation d'un produit, caractérisé en ce que le produit est destiné à l'hormonothérapie substitutive et contient un oestrogène et un progestatif sous la forme d'une préparation associée destinée à l'administration chez une femme nécessitant une hormonothérapie, de façon continue et consécutive, par alternance d'un certain nombre de doses unitaires à activité oestrogénique relativement dominante et d'un certain nombre de doses unitaires à activité progestatique relativement dominante, chacun desdits nombres de doses unitaires consistant en 1 à 5 doses unitaires.

23. Emploi selon la revendication 22, lequel est destiné à l'administration chez une femme en âge de procréer ou plus âgée dont la production ovarienne d'oestrogène et de progestérone a été interrompue soit à la suite d'une ménopause naturelle, soit à la suite d'une ablation ou extirpation ovarienne chirurgicale, par rayons ou par produits chimiques, soit à la suite d'une insuffisance ovarienne précoce.

24. Emploi selon la revendication 22 ou 23, dans lequel le produit présente les autres caractéristiques définies dans l'une quelconque des revendications 3 à 21.

## Revendications (Revendications pour l'(les) Etat(s) contractant(s) suivant(s): ES, GR)

1. Emploi d'un oestrogène et d'un progestatif dans un procédé de préparation d'un produit, caractérisé en ce que le produit est destiné à l'hormonothérapie substitutive et contient un oestrogène et un progestatif sous la forme d'une préparation associée destinée à l'administration, chez une femme nécessitant une hormonothérapie, de façon continue et consécutive, par alternance d'un certain nombre de doses unitaires à activité oestrogénique relativement dominante et d'un certain nombre de doses unitaires à activité progestative relativement dominante, chacun desdits nombres de doses unitaires consistant en 1 à 5 doses unitaires.

2. Emploi selon la revendication 1, dans lequel la femme à traiter est en âge de procréer ou plus âgée et sa production ovarienne d'oestrogène et de progestérone a été interrompue soit à la suite d'une ménopause naturelle, soit à la suite d'une ablation ou extirpation ovarienne chirurgicale, par rayons ou par produits chimiques, soit à la suite d'une insuffisance ovarienne précoce.

3. Emploi selon la revendication 1 ou 2, dans lequel chacun desdits nombres de doses unitaires consiste en 2 à 4 doses unitaires.

4. Emploi selon l'une quelconque des revendications précédentes, dans lequel chacun desdits nombres de doses unitaires consiste en 3 doses unitaires.

5. Emploi selon l'une quelconque des revendications précédentes, dans lequel les doses unitaires conviennent à l'administration par voie orale, parentérale, sublinguale, percutanée, intravaginale, intranasale ou buccale.

6. Emploi selon la revendication 5, dans lequel les doses unitaires conviennent à l'administration par voie orale.

7. Emploi selon la revendication 5, dans lequel les doses unitaires conviennent à l'administration par voie percutanée.

8. Emploi selon l'une quelconque des revendications précédentes, dans lequel l'oestrogène est sélectionné parmi des oestrogènes de synthèse sélectionnés parmi le 17α-éthinylestradiol et ses esters et éthers, l'éthinylestradiol, le mestranol et quinestranol et parmi des oestrogènes naturels sélectionnés parmi les oestrogènes équins conjugués, le 17β-estradiol, le valérate d'estradiol, l'estrone, le sulfate d'estrone, le sulfate d'estrone-pipérazine, l'estriol, le succinate d'estriol et le phosphate de polyestrol.

9. Emploi selon la revendication 8, dans lequel l'oestrogène est sélectionné parmi le sulfate d'estrone, le sulfate d'estrone-pipérazine et le 17β-estradiol.

10. Emploi selon l'une quelconque des revendications précédentes, dans lequel le progestatif est sélectionné parmi la progestérone, les esters de 17-hydroxyprogestérone, les esters de 19-nor-17-hydroxyprogestérone, la 17α-éthinyltestostérone et ses dérivés, la 17α-éthinyl-19-nortestostérone et ses dérivés, la noréthistérone, l'acétate de noréthistérone, le diacétate d'éthynodiol, la dydrogestérone, l'acétate de médroxyprogestérone, le noréthynodrel, l'allylestrénol, le lynestrénol, l'acétate de quingestanol, la médrogestone, la norgestriénone, la diméthistérone, l'éthistérone, la cyprotérone, l'acétate de cyprotérone, le lévo-norgestrel, le d-norgestrel, le dl-norgestrel, le d-17α-acétoxy-13β-éthyl-17α-éthinylgon-4-ène-3-one oxime, le gestodène, le norgestimate et le désogestrel.

11. Emploi selon la revendication 10, dans lequel le progestatif est la noréthistérone ou le norgestimate ou la progestérone.

12. Emploi selon l'une quelconque des revendications précédentes, dans lequel la quantité d'oestrogène par dose unitaire est comprise entre un minimum d'environ 0,3 mg et un maximum d'environ 5,0 mg de sulfate d'estronepipérazine ou une dose équivalente d'un autre oestrogène synthétique ou naturel et la quantité de progestatif par dose unitaire peut être comprise entre un minimum d'environ 0,0 mg et un maximum d'environ 5,0 mg de noréthistérone ou son équivalent en progestatif synthétique ou naturel.

13. Emploi selon la revendication 12, dans lequel la quantité d'oestrogène par dose unitaire est comprise entre 0,3 mg et 2,5 mg de sulfate d'estrone-pipérazine ou son équivalent.

14. Emploi selon la revendication 12 ou 13, dans lequel les doses unitaires sont destinées à l'administration orale et chacun desdits nombres est 1, 2, 3 ou 4 et les doses unitaires de l'association à activité oestrogénique relativement dominante contiennent 0,75 mg de sulfate d'estrone-pipérazine et les doses unitaires de l'association à activité progestative relativement dominante contiennent 0,75 mg de sulfate d'estrone-pipérazine et 0,35 mg de noréthistérone.

15. Emploi selon la revendication 12 ou 13, dans lequel les doses unitaires sont destinées à l'administration orale et chacun desdits nombres est 1, 2, 3 ou 4 et dans lequel les doses unitaires de l'association à dominance oestrogénique contiennent 0,75 mg de sulfate d'estrone-pipérazine et 0,15 mg de noréthistérone et les doses unitaires de l'association à dominance progestative contiennent 0,75 mg de sulfate d'estrone-pipérazine et 0,35 mg de noréthistérone.

16. Emploi selon la revendication 14 ou 15, dans lequel les doses unitaires sont destinées à l'administration en groupes alternants de 3 pour chaque type de dose unitaire contenant une hormone.

17. Emploi selon la revendication 12 ou 13, dans lequel chacun desdits nombres est 3 et dans lequel les doses de l'association à activité oestrogénique relativement dominante contiennent 1 mg de 17β-estradiol et les doses de l'association à activité progestative relativement dominante contiennent 1 mg de 17β-estradiol et 0,35 mg de noréthistérone, l'une et l'autre étant administrées par voie orale.

18. Emploi selon la revendication 12 ou 13, dans lequel chacun desdits nombres est 3 et dans lequel la dose unitaire de l'association à activité oestrogénique relativement dominante contient 0,75 mg de sulfate d'estrone et la dose unitaire de l'association à activité progestative relativement dominante contient 0,75 mg de sulfate d'estrone et 0,050 mg de norgestimate, l'une et l'autre étant administrées par voie orale.

19. Emploi selon la revendication 12 ou 13, dans lequel chacun desdits nombres est 3 et dans lequel la dose unitaire de l'association à activité oestrogénique relativement dominante contient 1,25 mg de sulfate d'estrone et l'association à dominance progestative relative contient 1,25 mg de sulfate d'estrone et soit 0,35 mg, soit 0,5 mg de noréthistérone, l'une et l'autre étant administrées par voie orale.

20. Emploi selon la revendication 12 ou 13, dans lequel chacun desdits nombres est 1, 2, 3 ou 4 et dans lequel l'association à activité oestrogénique relativement dominante contient 0,75 mg de sulfate d'estrone et 0,15 mg de noréthistérone et l'association à activité progestative relativement dominante contient 0,75 mg de sulfate d'estrone et 0,35 mg de noréthistérone, l'une et l'autre étant administrées par voie orale.

21. Emploi selon la revendication 12 ou 13, dans lequel les doses unitaires sont destinées à l'administration percutanée et à l'administration en alternance par périodes de 3 jours, de 17β-estradiol à raison de 0,1 mg/jour et, par périodes de 3 jours, de 17β-estradiol à raison de 0,1 mg/jour et de noréthistérone à raison de 0,35 mg/jour.

22. Procédé de préparation d'un produit, caractérisé en ce que le produit est destiné à l'hormonothérapie substitutive et contient un oestrogène et un progestatif sous la forme d'une préparation associée destinée à l'administration chez une femme nécessitant une hormonothérapie, de façon continue et consécutive, par alternance d'un certain nombre de doses unitaires à activité oestrogénique relativement dominante et d'un certain nombre de doses unitaires à activité progestative relativement dominante, chacun desdits nombres de doses unitaires consistant en 1 à 5 doses unitaires.

23. Procédé de préparation d'un produit de substitution hormonale comprenant un oestrogène et un progestatif, comprenant les étapes consistant à sélectionner une quantité d'oestrogène et une quantité de progestatif appropriées pour obtenir une dose unitaire à activité oestrogénique relativement dominante, à mélanger l'oestrogène et le progestatif de la dose à activité oestrogénique relativement dominante avec un excipient pharmaceutique, à sélectionner une quantité d'oestrogène et de progestatif appropriée pour obtenir une dose unitaire à activité progestative relativement dominante, à mélanger l'oestrogène et le progestatif de la dose à activité progestative relativement dominante avec un excipient pharmaceutique et à conditionner les doses unitaires selon une disposition destinée à l'administration continue et consécutive, par alternance, d'un certain nombre de doses unitaires à activité oestrogénique relativement dominante et d'un certain nombre de doses unitaires à activité progestative relativement dominante, chacun desdits nombres de doses unitaires consistant en 1 à 5 doses unitaires.

24. Procédé selon la revendication 23, dans lequel les doses unitaires sont moulées pour former des comprimés et les comprimés sont présentés en un conditionnement transparent comprenant 28 doses unitaires disposées selon un ordre fixe.

25. Procédé selon la revendication 24, dans lequel le conditionnement comprend des indications de dates adjacentes à chaque comprimé, indiquant le jour auquel le comprimé correspondant doit être pris.

26. Procédé selon l'une quelconque des revendications 22 à 25, dans lequel le produit est destiné à l'administration chez une femme en âge de procréer ou plus âgée dont la production ovarienne d'oestrogène et de progestérone a été interrompue soit à la suite d'une ménopause naturelle, soit à la suite d'une ablation ou extirpation ovarienne chirurgicale, par rayons ou produits chimiques, soit à la suite d'une insuffisance ovarienne précoce.

27. Procédé selon l'une quelconque des revendications 22 à 26, lequel possède les caractéristiques supplémentaires définies dans l'une quelconque des revendications 3 à 21.
